# EUROPEAN PATENT APPLICATION

(11) **EP 1 767 541 A1**
(43) Date of publication of application: **28.03.2007**
(21) Application number: 05743765.9
(22) Date of filing: 25.05.2005
(51) Int. Cl.: C07K 7/06, A61K 39/00, A61P 19/00, A61P 35/00, A61P 35/04, C07K 14/47, C12N 5/06

(54) **HLA-A24- OR HLA-A2-BINDING PEPTIDE OF PARATHYROID HORMONE-RELATED PROTEIN**

(30) Priority: 26.05.2004 JP 2004156460
(71) Applicant: Green Peptide Co., Ltd., Kurume-shi, Fukuoka 8300018 (JP)
(72) Inventor: ITOH, Kyogo, Miyaki-gun, Saga 8410205 (JP); HARADA, Mamoru, Fukuoka-shi, Fukuoka 8130011 (JP)
(74) Representative: Vossius & Partner
(86) International application number: PCT/JP2005/009542
(87) International publication number: WO 2005/116056

(57) **Abstract**

A cancer antigen peptide which is a partial peptide derived from parathyroid hormone-related protein, and which is capable of binding to an HLA-A24 or HLA-A2 antigen and being recognized by cytotoxic T lymphocytes, or a derivative thereof having the functionally equivalent properties, can be useful for the treatment and prevention of prostate cancer.

## Description

### TECHNICAL FIELD

The invention relates to identification of parathyroid hormone-related protein-derived antigen peptides immunogenic in human histocompatibility leukocyte antigen (HLA) -A24⁺ or HLA-A2⁺ prostate cancer patients, and particularly relates to specific cancer antigen peptides for the treatment or prevention of prostate cancer, cancer vaccines comprising the same, and the therapeutic methods therefor.

### BACKGROUND OF THE INVENTION

Prostate cancer is one of the most common cancers among elder men [1]. Prostate cancer frequently metastasizes to bone, and androgen blockade therapy has been applied for patients with bone metastases. Although the hormone therapy can temporarily inhibit the progress of the disease in such patients, the progression to hormone-refractory prostate cancer is inevitable in most cases. Therefore, the development of new therapeutic modalities is needed.

Recent advances in tumor immunology have allowed us to identify genes encoding human cancer-related antigens, and their antigenic epitopes that are recognized by cytotoxic T lymphocytes (CTLs), in patients with various types of cancers [2 - 4]. These identified cancer antigens and their peptides have been applied for specific immunotherapy [5 - 7]. In the case of prostate cancer, tissue-specific antigens, which are expressed in the normal prostate, can also be target molecules for specific immunotherapy for patients with this disease. Immunotherapy targeting prostate-specific antigen (PSA) or prostate-specific membrane antigen (PSMA) has been carried out, and anti-tumor effects have been observed in limited cases [8 - 12].

Parathyroid hormone-related protein (PTHrP) binds to receptors on osteoblasts, and stimulates bone formation and reabsorption. PTHrP has limited homology with Parathyroid hormone (PTH) at its NH₂ terminus and can bind to the same receptor as PTH, resulting in similar biological activity [13]. PTHrP plays a variety of physiological roles, including calcium transport, keratinocyte differentiation, smooth muscle relaxation, and cartilage development [14]. In parathyroid cells, a high extracellular calcium concentration inhibits parathyroid hormone (PTH) secretion and proliferation of parathyroid cells as a result of negative feedback regulation, whereas it evokes further PTHrP secretion and promotes worsening bone resorption [15]. Therefore, PTHrP has been regarded to be responsible for the hypercalcemia associated with malignancy [16]. In addition, prostate cancers have been reported to produce PTHrP [17]. These lines of evidence suggest that PTHrP could be a promising target molecule for immunotherapy of prostate cancer patients with bone metastases. PTHrP 59-68 and PTHrP 165-173 peptides have been reported to be candidates for such specific immunotherapeutic treatment of HLA-A2⁺ prostate cancer patients [16, 17].
In this study, the inventors attempted to identify novel PTHrP-derived peptides which have immunogenicity in HLA-A24⁺ and A2⁺ prostate cancer patients.

### DISCLOSURE OF THE INVENTION

### PROBLEM TO BE SOLVED BY THE INVENTION

An object of the present invention is to provide a novel, immunogenic PTHrP-derived peptide. Another object of the present invention is to provide a specific cancer antigen peptide, a pharmaceutical composition comprising the peptide, and a therapeutic method using the peptide.

### MEANS FOR SOLVING THE PROBLEM

Thus, the present invention provides:
(1) A cancer antigen peptide which is a partial peptide derived from parathyroid hormone-related protein, and which is capable of binding to an HLA-A24 or HLA-A2 antigen and being recognized by cytotoxic T lymphocytes, or a derivative thereof having the functionally equivalent properties; preferably, the cancer antigen peptide which is further recognized by humoral immune system or derivative thereof having the functionally equivalent properties;
(2) A cancer antigen peptide which comprises an amino acid sequence shown in any one of SEQ ID NOS: 1 to 9, or a derivative thereof which is capable of binding to HLA-A24 or HLA-A2 antigen and being recognized by cytotoxic T lymphocytes;
(3) A pharmaceutical composition for treating or preventing prostate cancer, which comprises as an active ingredient the cancer antigen peptide or derivative thereof according to (1) or (2) above, particularly the pharmaceutical composition which is preferably used when the prostate cancer is associated with bone metastases;
(4) A pharmaceutical composition for treating or preventing metastatic bone lesion, which comprises as an active ingredient the cancer antigen peptide or derivative thereof according to (1) or (2) above, particularly the pharmaceutical composition which is preferably used when the metastatic bone lesion are accompanied by prostate cancer;
(5) A cytotoxic T lymphocyte which specifically recognizes a complex between an HLA-A24 or HLA-A2 antigen and the cancer antigen peptide or derivative thereof according to (1) or (2) above;
(6) A pharmaceutical composition for treating prostate cancer, which comprises as an active ingredient the cytotoxic T lymphocyte of (5) above, particularly, the pharmaceutical composition which is preferably used when the prostate cancer is associated with bone metastases;
(7) An antigen presenting cell which presents a complex between HLA-A24 or HLA-A2 antigen and the cancer antigen peptide or derivative thereof according to (1) or (2) above on the cell surface;
(8) A method for treating or preventing prostate cancer, which comprises administering to a patient in need thereof the cancer antigen peptide or derivative thereof according to (1) or (2) above;
(9) A method for treating or preventing metastatic bone lesion, which comprises administering to a patient in need thereof the cancer antigen peptide or derivative thereof according to (1) or (2) above.
(10) Use of the cancer antigen peptide or derivative thereof according to (1) or (2) above for manufacturing a pharmaceutical composition for treating or preventing prostate cancer; and
(11) Use of the cancer antigen peptide or derivative thereof according to (1) or (2) above for manufacturing a pharmaceutical composition for treating or preventing metastatic bone lesion.

The term "Parathyroid hormone-related protein" abbreviated as PTHrP means a family of protein hormones produced by most if not all tissues in the body. PTHrP is encoded by a single gene that is highly conserved among species, and the amino acid sequence of PTHrP is described in Reference (35). The cancer antigen peptide of the present invention can be identified by synthesizing a candidate peptide which comprises a part of PTHrP and conducting an assay for determining whether or not a complex between the candidate peptide and an HLA molecule is recognized by CTL. For example, identification of the cancer antigen peptide of the present invention can be conducted according to the illustration of the working examples hereinafter. In the invention, PTHrP peptides which are effectively recognized by both the humoral and cellular immune systems in HLA-A24⁺ or HLA-A2⁺ prostate cancer patients are particularly preferred.
The present invention provides the peptide which comprises an amino acid sequence shown in any one of SEQ ID NOS: 1 to 9. Particularly, as the cancer antigen peptide of the present invention, the peptide which comprises the amino acid sequence shown in any one of SEQ ID NOS: 1, 2, 5 and 6 is preferably used.

The cancer antigen peptide of the present invention preferably consists of 8 to 50, more preferably 8, 9, 10, or 11 amino acid residues.
Synthesis of the peptides may be conducted according to methods usually used in peptide chemistry. Examples of the known methods are those described in the literatures including "Peptide Synthesis", Interscience, New York, 1966; "The Proteins", vol. 2, Academic Press Inc., New York, 1976; "Pepuchido-Gosei", Maruzen Co. Ltd., 1975.
As used herein, "derivative of a cancer antigen peptide" means a peptide whose amino acid sequence contains substitution, deletion and/or addition of one or two amino acid residues compared to that of the cancer antigen peptide. "A derivative of the cancer antigen peptide having the functionally equivalent properties" means a peptide which is derived from the cancer antigen peptide and has the properties as a cancer antigen peptide, i.e., is capable of binding to an HLA-A24 or HLA-A2 antigen and being recognized by CTL.

At least one of, or a combination of two or more of, the cancer antigen peptides or derivatives thereof of the present invention is (are) administered to a patient, if necessary, in combination with other anti-cancer agents such as immunomodulators and chemotherapeutics. When administered, the cancer antigen peptide or derivative thereof is presented at a high density with an HLA-A24 antigen of an antigen-presenting cell, and therefore, a CTL specific for the presented HLA antigen complex proliferates and destroys cancer cells. As a result, the cancer of the patient may be treated, or proliferation or metastasis of the cancer may be prevented.

The cancer antigen peptide or derivative thereof of the present invention can be used for a pharmaceutical composition for treating or preventing prostate cancer. The present invention, therefore, provides a pharmaceutical composition for treating or preventing prostate cancer, which comprises, as an active ingredient, one, two, or more of the cancer antigen peptides or derivatives thereof of the present invention. The pharmaceutical composition of the invention is particularly useful when the prostate cancer is associated with bone metastases. The cancer antigen peptide or derivative thereof of the present invention also can be used for a pharmaceutical composition for treating or preventing metastatic bone lesion, and it is preferably used when the metastatic bone lesion are accompanied by prostate cancer. The invention is based on the fact that prostate cancer is highly prone to bone metastasis and produces PTHrP involved in bone resorption, and the result that the PTHrP peptide of the present invention can induce a prostate cancer-reactive CTL.

The composition comprising as an active ingredient the cancer antigen peptide or derivative thereof of the present invention may be administered intradermally or hypodermically. The composition of the invention may be administered along with an adjuvant in order to effectively establish cellular and/or humoral immunity. Although the amount of the cancer antigen peptide or derivative thereof of the present invention to be administered in the formulation may be adjusted as appropriate depending on, for example, condition of the disease to be treated, and age, body weight, and the like of the particular patient, it is usually 0.1 mg to 10.0 mg, preferably 0.5 mg to 5.0 mg, more preferably 1.0 mg to 3.0 mg, for every several days, one or two to several weeks, or one or two to several months.

The present invention further provides a method for treating or preventing prostate cancer or metastatic bone lesion, which comprises administering to a patient in need thereof the cancer antigen peptide of the present invention or derivative thereof having the functionally equivalent properties.

The present invention provides a CTL that specifically recognizes a complex between an HLA-24 or HLA-A2 antigen and the cancer antigen peptide or derivative thereof, and also provides a pharmaceutical composition for treating prostate cancer which comprises the CTL as an active ingredient. The composition of the invention preferably contains physiological saline or phosphate buffered saline (PBS). It may be administered, for example, intravenously or at the site of the cancer.

Additionally, the present invention provides an antigen presenting cell which presents a complex between HLA-A24 or HLA-A2 antigen and the cancer antigen peptide or derivative thereof on the cell surface. The antigen presenting cell of the invention can be prepared by administering the peptide or derivative thereof of the invention to a cell having antigen-presenting capability which is positive for the corresponding HLA antigen to present a complex between the peptide or derivative and the HLA antigen on the cell surface. When administered to a patient, the antigen presenting cell of the invention can induce a CTL which specifically recognizes the complex between the peptide or derivative administered and the HLA antigen within the body of the patient.

### BRIEF DESCRIPTION OF THE DRAWINGS

Fig. 1: The results of flow cytometric analysis for LNCaP and an HLA-A24-expressing LNCaP cell line, LNCaP-A24. The dotted lines represent staining without the first mAb.
Fig. 2: Induction of HLA-A24-restricted and prostate cancer-reactive CTLs from PBMCs of healthy donors (HD) and cancer patients (Pt).
   (A) Graphs showing inductions of prostate cancer-reactive CTLs by indicated peptides, and their cytotoxicity against LNCaP cells (HLA-A24-), LNCaP-A24 cells (HLA-A24⁺), and PHA-blastoid T cells (HLA-A24⁺). Values represent the mean of triplicate assays. * P<0.05 was considered statistically significant.
   (B) Graphs showing inhibition of cytotoxicity of PTHrP peptide-stimulated PBMCs with antibodies. Values represent the mean of triplicate assays. * P<0.05 was considered statistically significant.
   (C) Graphs showing inhibition of cytotoxicity of PTHrP peptide-stimulated CTLs. Peptide-pulsed, unlabelled T2 cells were used as cold target cells. Values represent the mean of triplicate assays. * P<0.05 was considered statistically significant.

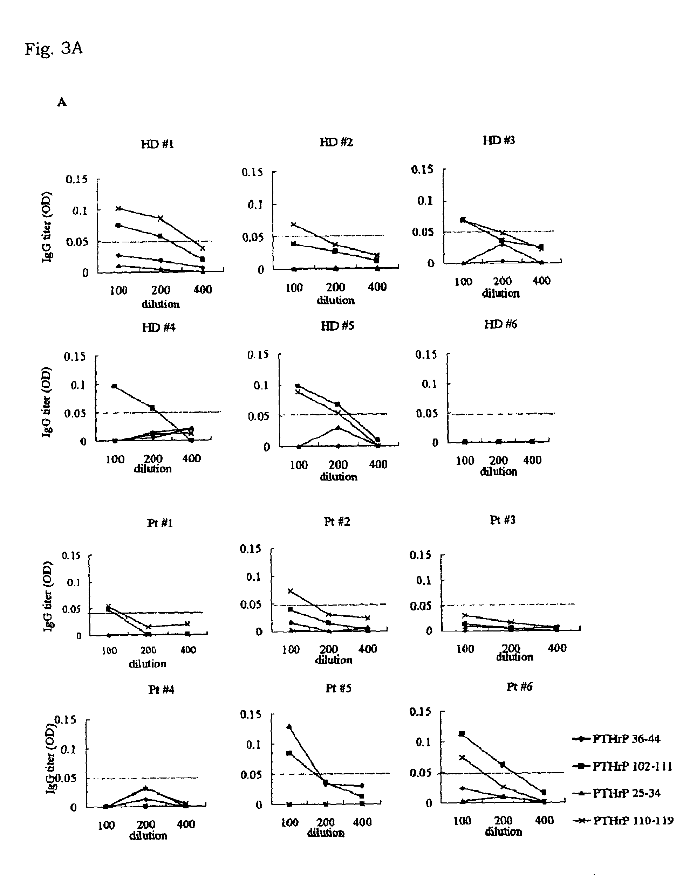

Fig. 3: Reactivity of IgGs in plasma from healthy donors and prostate cancer patients to PTHrP peptides.
   (A) Graphs showing the results of peptide-specific IgGs from six healthy donors and six prostate cancer patients expressed as optical density (OD).
   (B) Graphs showing peptide-specific absorption of IgGs to the indicated PTHrP peptides.

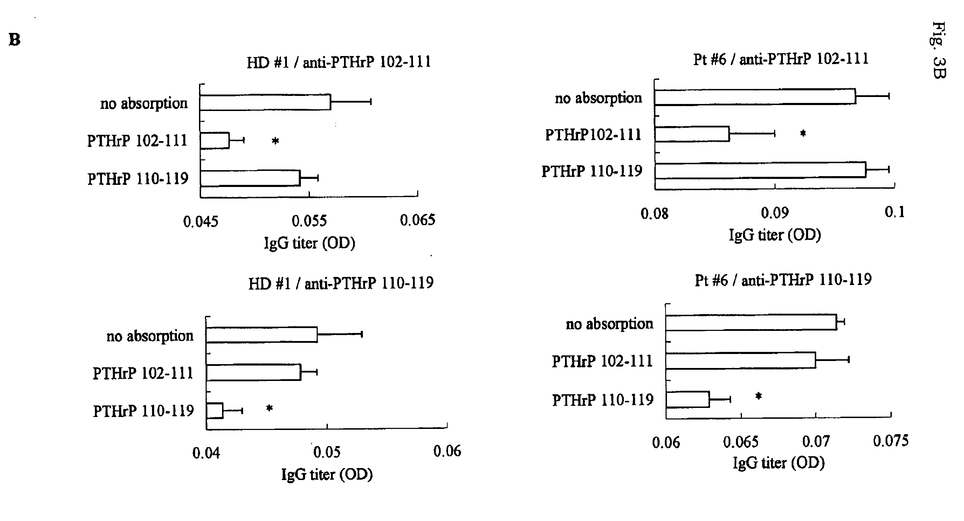

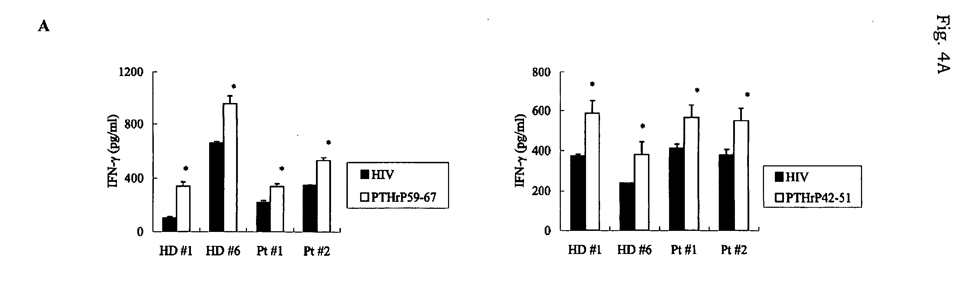

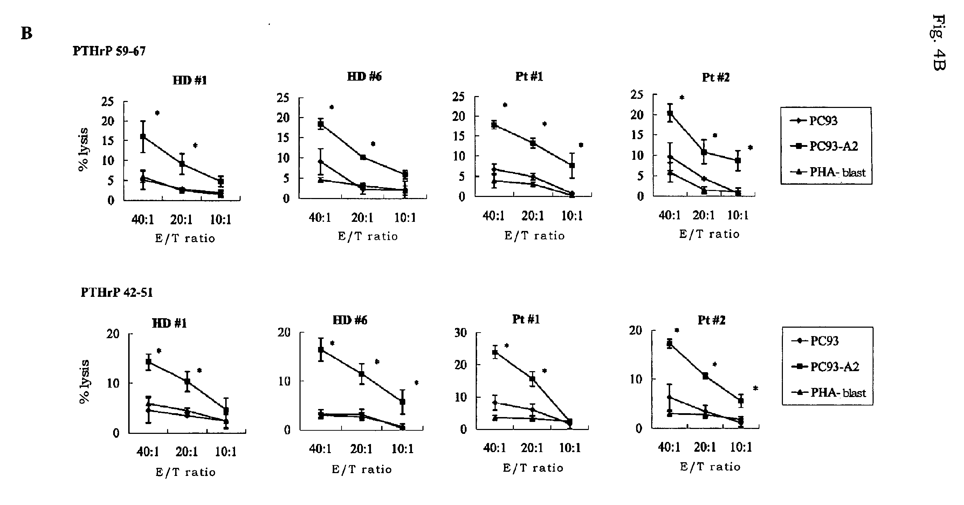

Fig. 4: Induction of HLA-A2-restricted and prostate cancer-reactive CTLs from PBMCs of healthy donors and cancer patients.
   Graphs showing (A) induction of peptide-specific CTLs by the indicated peptides, and (B) their cytotoxicity against PC93 cells (HLA-A2-), PC93 -A2 cells (HLA-A2⁺), and PHA-blastoid T cells (HLA-A2⁺). Values represent the mean of triplicate assays. * P<0.05 was considered statistically significant.

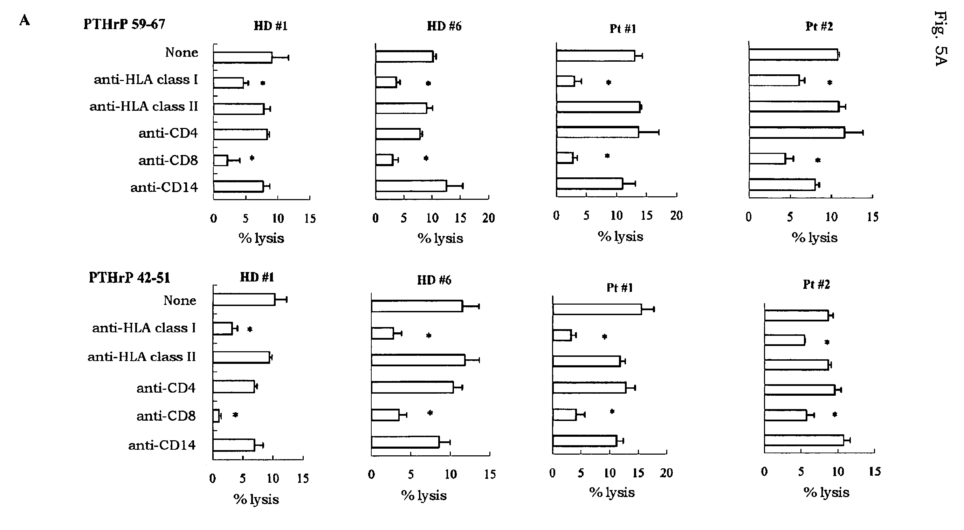

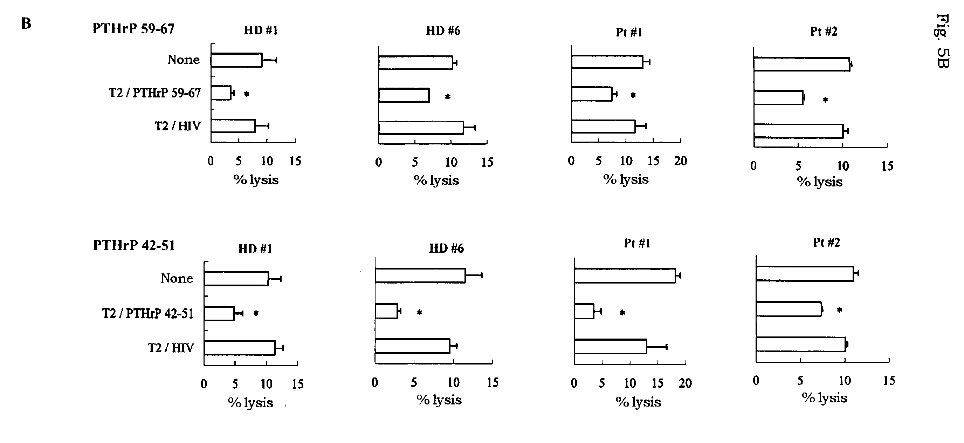

Fig. 5: (A) Graphs showing inhibition of cytotoxicity of PTHrP peptide-stimulated PBMCs with antibodies. Values represent the mean of triplicate assays. * P<0.05 was considered statistically significant.
   (B) Graphs showing inhibition of cytotoxicity of PTHrP peptide-stimulated CTLs. Peptide-pulsed, unlabelled T2 cells were used as cold target cells. Values represent the mean of triplicate assays. * P<0.05 was considered statistically significant.

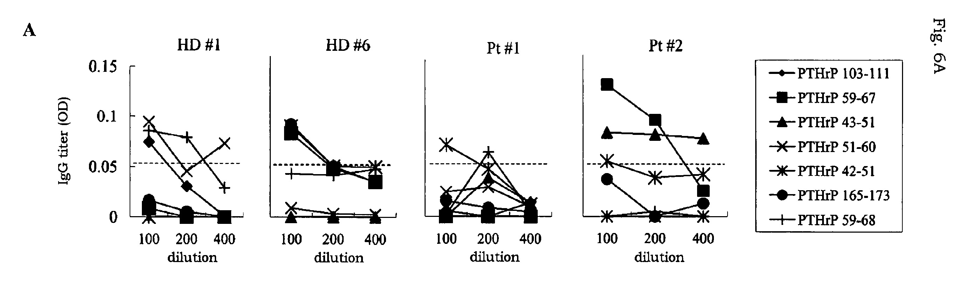

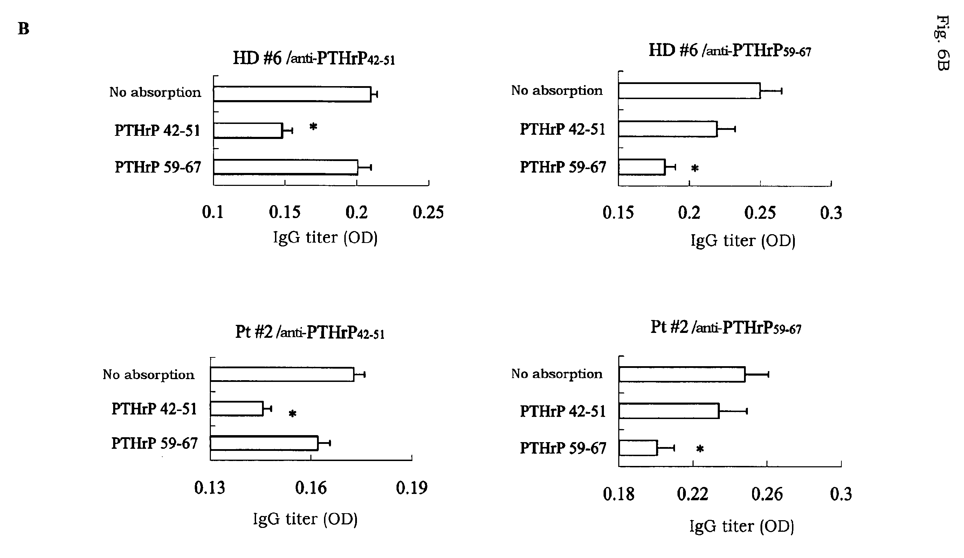

Fig. 6: Reactivity of IgGs in plasma from healthy donors and prostate cancer patients to PTHrP peptides.
   (A) Graphs showing the results of peptide-specific IgGs from two healthy donors and two prostate cancer patients expressed as optical density (OD).
   (B) Graphs showing peptide-specific absorption of IgGs which recognize the indicated PTHrP peptides.

### DETAILED DESCRIPTION OF THE PREFERRED EMBODIMENTS

Prostate cancer appears to be a good target for the development of specific immunotherapies [25]. In recent years, the inventors have attempted to identify epitope peptides derived from prostate-related antigens that would be able to generate prostate cancer-reactive CTLs from prostate cancer patients [22, 23, 26]. However, one major obstacle encountered when treating prostate cancer patients is the treatment of prostate cancer metastasized to bone tissues. Therefore, the inventors undertook the present study to identify antigenic epitopes which could potentially be suitable for specific immunotherapeutic treatment of HLA-A24⁺ or HLA-A2⁺ prostate cancer patients with metastases.

In this study, PTHrP-derived peptides immunogenic in HLA-A24⁺ or HLA-A2⁺ prostate cancer patients were identified. PTHrP 36-44 and PTHrP 102-111 peptides, among four different PTHrP peptides carrying the HLA-A24 binding motif, and PTHrP 42-51 and PTHrP 59-67 peptides, among seven different PTHrP peptides carrying the HLA-A2 binding motif, efficiently induced peptide-specific cytotoxic T lymphocytes from peripheral blood mononuclear cells (PBMCs) of HLA-A24⁺ and HLA-A2⁺ prostate cancer patients, respectively. Peptide-stimulated PBMCs showed cytotoxicity against prostate cancer cells in an HLA-A24 or HLA-A2-restricted manner. Experiments using antibodies and cold inhibition targets confirmed that their cytotoxicity was dependent on PTHrP peptide-specific and CD8⁺ T cells. Further, immunoglobulin Gs (IgGs) reactive to the PTHrP 102-111 or the PTHrP 110-119 peptide which binds to an HLA-A24 molecule and PTHrP 42-51 peptide which binds to an HLA-A2 molecule were frequently detected in the plasma of prostate cancer patients, suggesting that the PTHrP 102-111 and PTHrP 42-51 peptides are efficiently recognized by both cellular and humoral immune responses in cancer patients. These results indicate that the peptide of the invention could be a promising target molecule for specific immunotherapy of HLA-A24⁺ or HLA-A2⁺ prostate cancer patients with metastases.

In the present invention, new PTHrP peptides which have the potential to generate prostate cancer-specific CTLs in HLA-A24⁺ or HLA-A2⁺ prostate cancer patients have been identified, and thereby the possibility of creating a peptide vaccine targeting PTHrP has been expanded. The inventors revealed that both the PTHrP 36-44 and PTHrP 102 -111 peptides have the potential to more efficiently induce prostate cancer-reactive CTLs in HLA-A24⁺ prostate cancer patients, as well as the PTHrP 42-51 and PTHrP 59-67 peptides in HLA-A2⁺ prostate cancer patients. PBMCs from HLA-A24⁺ prostate cancer patients showed peptide-specific IFN-γ production in 7 or 6 of 10 patients when stimulated with the PTHrP 36-44 peptide or the PTHrP 102-110 peptide, respectively. HLA-A2-binding PTHrP 42-51 and the PTHrP 59-67 peptides induced peptide-specific IFN-γ production in 5 or 4 of 10 patients, respectively. More importantly, PBMCs that were stimulated with these PTHrP peptides showed cytotoxicity against prostate cancer cells in an HLA-A24- or HLA-A2-restricted manner. These results indicate that the above PTHrP peptides are immunogenic and therefore potentially useful for specific immunotherapy of HLA-A24⁺ or HLA-A2⁺ prostate cancer patients with metastases.

Those peptides also induced peptide-specific and cancer-reactive CTLs from the PBMCs of HLA-A24⁺ or HLA-A2⁺ healthy donors. This result is consistent with that of a previous report demonstrating induction of PTHrP peptide-specific CTLs from PBMCs of HLA-A2⁺ healthy donors [17]. As the homology between each of these PTHrP peptides and PTH is low, cross-reactivity between the PTHrP peptides and PTH could be excluded. Low levels of PTHrP have been sporadically detected in keratinocytes, uterus, and mammary glands during lactation [27]. Recent advances in tumor immunology have revealed that self-antigens on human cancer cells are the most prevalent antigens recognized by the immune system [2-4]. CTL precursors reactive to non-mutated self antigens might circulate in the peripheral blood of both certain healthy donors and cancer patients.

Here, the inventors investigated whether or not IgGs against PTHrP peptides would be detectable in the plasma from HLA-A24⁺ or HLA-A2⁺ prostate cancer patients, because antibodies against class 1-binding cancer antigen peptides had already been observed in certain cancer patients and healthy donors [20, 21]. We also previously reported that IgGs reactive to peptides derived from prostate-related antigens were frequently detectable in healthy donors and prostate cancer patients [22-24]. In the present invention, IgGs reactive to either the PTHrP 102-111 or PTHrP 110-119 peptide among the HLA-A24-binding peptides and the PTHrP 42-51 peptide among the HLA-A2-binding peptides were frequently detected in healthy donors as well as in prostate cancer patients. This means that the PTHrP 102-111 and PTHrP 42-51 peptides were efficiently recognized by both the cellular and the humoral immune system. Although the roles played by peptide-specific IgG in anti-tumor immune responses have not been clear yet, the clinical trials conducted by the inventors revealed that peptide vaccination frequently resulted in the induction of IgGs reactive to administered peptides [28, 29]. In addition, the induction of IgGs reactive to vaccinated peptides was positively correlated with longer survival of advanced lung cancer patients [30]. As regards the use of peptide vaccination in cases of gastric cancer, prolonged survival has been observed in patients showing not only cellular but also humoral immune responses to vaccinated peptides [31]. Furthermore, the induction of IgGs reactive to the administered peptides was correlated with a clinical response among patients with recurrent gynecologic cancer [32]. Interestingly, the levels of IgG specific to the PTHrP 102-111 peptide was lower in patients with advanced HRPC than in those with non-advanced prostate cancer (unpublished data). Vaccination with the PTHrP 102-111 peptide into such patients could elicit the induction of peptide-specific IgG and might lead to clinical responses. Further study could shed light on the role of peptide-specific IgG in the anti-tumor immune response.

### EXAMPLES

The present invention is further illustrated by the following examples, but is not limited by these examples in any respect. The statistical significance of the data was determined using a two-tailed Student's t-test. A P value of less than 0.05 was considered to be statistically significant.

### Example 1

### Identification of candidates to generate peptide-specific CTLs from HLA-A24⁺ prostate cancer patients

### 1.1 Patients

All HLA-A24⁺ prostate cancer patients and HLA-A24⁺ healthy donors were enrolled in this study after informed consent was obtained. The prostate cancer patients #2, #3, #5, and #9 were associated with bone metastases. None of these participants was infected with HIV. Twenty milliliters of peripheral blood was obtained, and PBMCs were prepared by Ficoll-Conray density gradient centrifugation. The expression of HLA-A24 molecules on the PBMCs of cancer patients and healthy donors was determined by flow cytometry.

### 1.2 Cell lines

C1R-A24 is an HLA-A*2402-expressing subline of C1R lymphoma (Oiso M, Eura M, Katsura F, Takiguchi M, Sobao Y, Masuyama K, Nakashima M, Itoh K, Ishikawa T. A newly identified MAGE-3-derived epitope recognized by HLA-A24-restricted cytotoxic T lymphocytes. Int. J. Cancer 81: 387-394, 1999). All cell lines were maintained in RPMI-1640 medium (Gibco BRL, Grand Island, NY) supplemented with 10% FCS.

### 1.3 Peptides

Four PTHrP-derived peptides (listed in Table 1 hereinafter) were prepared based on their binding affinity to HLA-A24 molecules, HLA-A24 binding motif [18, 19]. All peptides were of >90% purity and were purchased from Biologica Co., Nagoya, Japan. Influenza (Flu) virus-derived (RFYIQMCYEL), EBV-derived (TYGPVFMCL), and HIV-derived peptides (RYLRQQLLGI) with the HLA-A24 binding motif were used as controls. All peptides were dissolved with DMSO at a dose of 10 mg/ml.
Although the PTHrP 1-36 peptide is a propeptide [35], the PTHrP 25-34 peptide was included in the sequence. As regards the difference in amino acids, three amino acids were found to differ between the PTHrP 36-44 peptide and PTH, and all of the amino acids were found to differ between the other three PTHrP peptides and PTH.

### 1.4 Assay for peptide-specific CTLs in PBMCs

To investigate the immunogenicity of these four PTHrP peptides, the PBMCs of 10 HLA-A24⁺ healthy donors and 10 HLA-A24⁺ prostate cancer patients were stimulated with each of the four PTHrP peptides and were examined for their IFN-γ production in response to C1R-A24 cells, which were pre-pulsed with either a corresponding PTHrP peptide or the HIV peptide. Flu- and EBV-derived peptides were used as controls.

The assay for detection of peptide-specific CTLs in PBMCs was performed according to a previously reported method [34]. In brief, PBMCs (1 X 10⁵ cells/well) were incubated with 10 µg/ml of each peptide in a U-bottom-type 96-well microculture plate (Nunc, Roskilde, Denmark) at a volume of 200 µl of culture medium. The culture medium consisted of 45% RPMI-1640, 45% AIM-V medium (Gibco BRL), 10% FCS, 100 U/ml of IL-2, and 0.1 mM MEM nonessential amino acid solution (Gibco, BRL). Half of the culture medium was removed and replaced with new medium containing a corresponding peptide (20 µg/ml) every 3 days. On the 15th day of culture, the cultured cells were separated into 4 wells, and two wells were used for PTHrP peptide-pulsed C1R-A24 cells, and the other two wells were used for the HIV peptide-pulsed C1R-A24 cells. After an 18-hr incubation period, the supernatants were collected, and the level of IFN-γ was determined by ELISA (limit of sensitivity: 10 pg/ml).
The assay was carried out in quadruplicate, and the mean of two wells which show the highest significance compared to the control is shown in Table 1.

Table 1

**Table 1 Reactivity ofPTHrP peptide-stimulated PBMCs from HLA A24⁺ healthy donors and prostate cancer patients**

| PBMCs derived from | Peptides | | | | | | |
|---|---|---|---|---|---|---|---|
| | Name Amino acid sequence Score¹ | PTHrP 36-44 RAVSEHQLL 14.4 | PTHrP 102-111 RYLTQETNKV 19.8 | PTHrP 25-34 RSVEGLSRRL 17.3 | PTHrP 110-119 KVETYKEQPL 14.4 | Flu RFYIQMCTEL | EBV TYGPVFMCL |
| | IFN-γ production (pg/mL)² | | | | | | |
| Healthy #1 donors | | 154 | 352 | 10 | 394 | 306 | 0 |
| #2 | | 156 | 132 | 8 | 17 | 0 | 207 |
| #3 | | 497 | 0 | 7 | 20 | 17 | 0 |
| #4 | | 0 | 0 | 37 | 2 | 59 | 14 |
| #5 | | 184 | 0 | 166 | 38 | 0 | 27 |
| #6 | | 1354 | 0 | 0 | 357 | 124 | 168 |
| #7 | | 166 | 38 | 0 | 0 | 1017 | 0 |
| #8 | | 0 | 194 | 0 | 1017 | 0 | 0 |
| #9 | | 0 | 5624 | 5 | 61 | 123 | 228 |
| #10 | | 0 | 168 | 1354 | 0 | 0 | 3 |
| Total | | 6/10 | 5/10 | 2/10 | 3/10 | 4/10 | 3/10 |
| Cancer patients #1 | | 180 | 154 | 145 | 0 | 0 | 15 |
| #2 | | 122 | 138 | 15 | 9 | 5 | 0 |
| #3 | | 699 | 8 | 17 | 38 | 0 | 21 |
| #4 | | 31 | 105 | 24 | 19 | 0 | 159 |
| *#5* | | 799 | 28 | 16 | 10 | 130 | 20 |
| #6 | | 500 | 4 | 1 | 14 | 198 | 15 |
| #7 | | 317 | 0 | 0 | 0 | N.D. | N.D. |
| #8 | | 4 | 1060 | 411 | 23 | 115 | 189 |
| #9 | | 17 | 101 | 1 | 0 | 709 | 3 |
| #10 | | 180 | 198 | 196 | 118 | 40 | 27 |
| Total | | 7/10 | 6/10 | 3/10 | 1/10 | 4/9 | 2/9 |

| | | | | | | | |
|---|---|---|---|---|---|---|---|
| ¹ The score represents the estimated half-time of dissociation of the PTHrP peptides binding to HLA-A24 molecules. ² The PBMCs of HLA-A24⁺ healthy donors and prostate cancer patients were stimulated *in vitro* with the indicated PTHrP peptide, as described in Examples. On day 15, the cultured PBMCs were tested for their reactivity to C1R-A24 cells, which were pro-pulsed with a corresponding peptide or an HIV peptide. The values represent the mean of 2 wells, and the background IFN-γ production in response to the HIV peptide was subtracted. Significant values (p<0.05 by tw o-tailed Student's t-test) are underlined. N.D., not done. | | | | | | | |

Successful induction of peptide-specific CTLs was judged to be positive when significant values (P<0.05 by two tailed Student's t-test) were observed. As a result, the PTHrP 36-44, PTHrP 102-111, PTHrP 25-34, and PTHrP 110-119 peptides induced peptide-specific CTLs in 6, 5, 2, and 3 of 10 HLA-A24⁺ healthy donors, respectively. These PTHrP peptides also induced peptide-specific CTLs in 7, 6, 3, and 1 of 10 HLA-A24⁺ prostate cancer patients, respectively. These findings indicate that the PTHrP 36-44 and the PTHrP 102-111 peptides are, among others, promising candidate peptides to generate peptide-specific CTLs from HLA-A24⁺ prostate cancer patients.

### Example 2

### Induction of prostate cancer-reactive CTLs that are HLA-A24-restricted and specific for the PTHrP 36-44 or PTHrP 102-111 peptide

### 2.1 An HLA-A24-expressing prostate cancer cell line

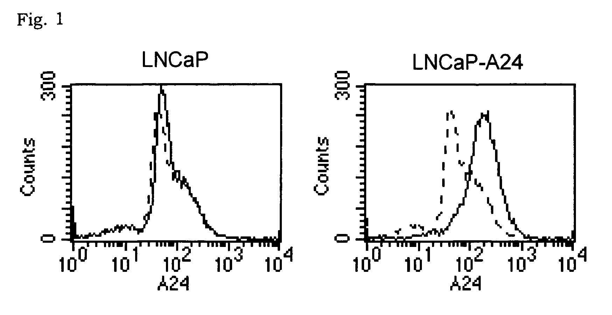

In order to investigate the HLA-A24-restricted and prostate cancer-reactive cytotoxicity of peptide-stimulated PBMCs, an HLA-A24-expressing LNCaP cell line, which was designated as LNCaP-A24, was prepared. The LNCaP is an HLA-A24 negative prostate cancer cell line. To establish LNCaP cells stably expressing HLA-A24 molecules (designated as LNCaP-A24), a pcDNA3.1/Hygro vector (Invitrogen, CA), which was inserted with the HLA-A*2402 gene, was electroporated into the LNCaP cell line (ATCC number: CRL-1740), and selection was carried out with hygromycin B (Invitrogen) at a dose of 170 µg/ml. All cell lines were maintained in RPMI-1640 medium (Gibco BRL, Grand Island, NY, USA) supplemented with 10% FCS. LNCaP has previously been reported to produce PTHrP (17). A parental LNCaP cell line was negative for the cell surface expression of HLA-A24 molecules, whereas the LNCaP-A24 cell line expressed HLA-A24 molecules on their cell surface. Flow cytometric analysis was performed on LNCaP and LNCaP-A24 cells. These cells were stained with anti-HLA-A24 mAb, followed by FITC-conjugated anti-mouse IgG mAb. The dotted lines represent staining without the first mAb. The results of flow cytometric analysis for LNCaP and LNCaP-A24 are shown in Fig. 1.

### 2.2 Induction of prostate cancer-reactive CTLs by indicated peptides

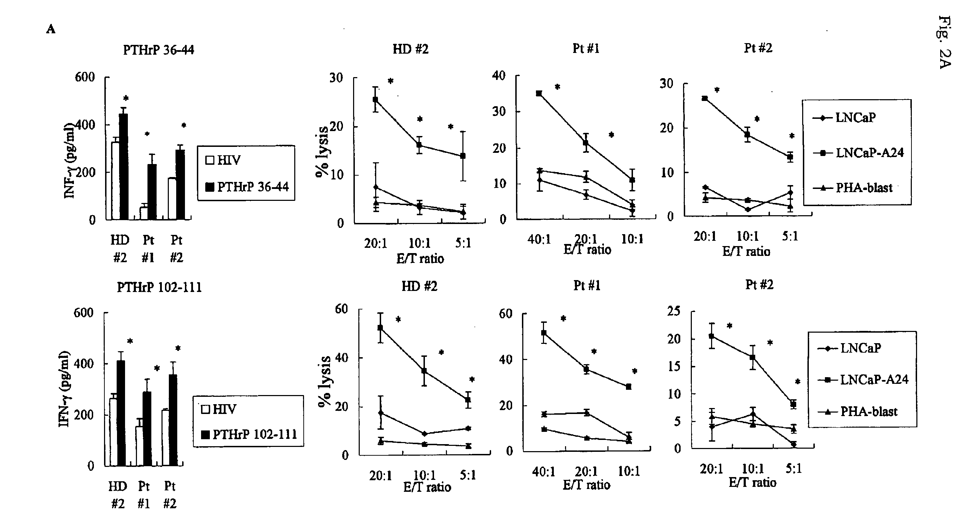

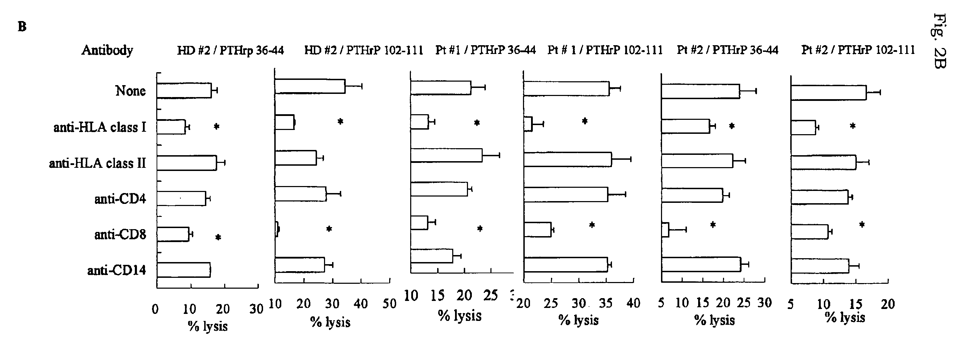

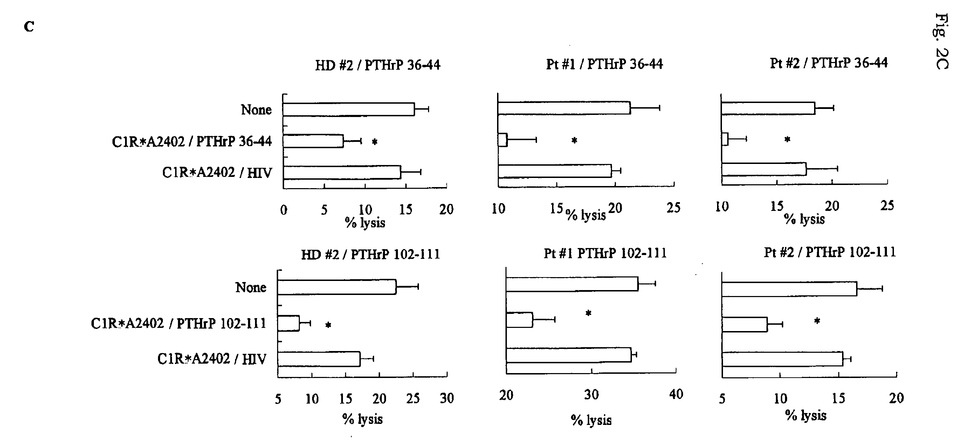

It was then determined whether PBMCs stimulated by either the PTHrP 36-44 or the PTHrP 102-111 peptide could induce prostate cancer-reactive CTLs from HLA-A24⁺ healthy donors and prostate cancer patients. PBMCs from HLA-A24⁺ healthy donors and prostate cancer patients were repeatedly stimulated with these PTHrP peptides. On day 15, half of the cultured cells were harvested, pooled from 4 wells, and cultured with C1R-A24 cells, which were pre-pulsed with the HIV peptide (open symbol) and the indicated PTHrP peptide (closed symbol) for 18-hr. The levels of IFN-γ in the supernatants were then determined by ELISA. As a result, the PTHrP peptide-stimulated PBMCs from HD#2, Pt#1, and Pt#2 produced higher levels of IFN-γ in response to the corresponding PTHrP peptide-pulsed C1R-A24 cells than to HIV peptide-pulsed C1R-A24 cells (Fig. 2A).

### 2.3 Cytotoxicity of peptide-stimulated PBMCs against three target cells

After confirmation that these peptide-stimulated cells could produce IFN-γ in response to PTHrP peptide-pulsed C1R-A24 cells, these peptide-stimulated PBMCs were examined for their cytotoxicity against three targets, LNCaP cells (HLA-A24-), LNCaP-A24 cells (HLA-A24⁺), and PHA-blastoid T cells (HLA-A24⁺). Specifically, after in vitro stimulation with the PTHrP peptides, the peptide-stimulated PBMCs were additionally cultured with 100 U/ml IL-2 for approximately 10 days, in order to obtain a sufficient number of cells to carry out a cytotoxicity assay. Then, the obtained cells were tested for cytotoxicity against both LNCaP and LNCaP-A24, and PHA-blastoid T cells by a 6-hr ⁵¹Cr-release assay. Two thousands of ⁵¹Cr-labeled cells per well were cultured with effector cells in 96-well round-bottomed plates at the indicated effector/target ratios. The results are also shown in Fig. 2A. The PTHrP peptide-stimulated PBMCs from HD#2, Pt#1, and Pt#2 also showed higher levels of cytotoxicity against the LNCaP-A24 cell line than against the LNCaP line and HLA-A24⁺ PHA-induced T cell blasts.

### 2.4 Inhibition of the cytotoxicity of PTHrP peptide-stimulated PBMCs by antibodies

Then, in some experiments, either of anti-HLA class I (W6/32: mouse IgG2a), anti-HLA-DR (L243: mouse IgG2a), anti-CD4 (NU-TH/I: mouse IgG1), anti-CD8 (NU-TS/C: mouse IgG2a), or anti-CD14 (H14: mouse IgG2a) mAb was added to the wells at a dose of 20 µg/ml of at the initiation of the assay. As a result, their cytotoxicity against the LNCaP-A24 was significantly inhibited by the addition of anti-HLA-class I or anti-CD8 mAbs, but not by the addition of other anti-HLA-class II, anti-CD4, or anti-CD 14 mAb (Fig. 2B).

### 2.5 Specificity of PTHrP peptide-stimulated PBMCs

Furthermore, the specificity of PTHrP peptide-stimulated PBMCs was confirmed by a cold inhibition assay. In brief, ⁵¹Cr-labbeled target cells (2 X 10³ cells/well) were cultured with the peptide-stimulated PBMCs (4 X 10⁴ cells/well) in 96-well round-bottomed plates with 2 X 10⁴ cold target cells. C1R-A24 cells, which were pre-pulsed with either the HIV peptide or a corresponding PTHrP peptide, were used as cold targets. Their cytotoxicity against the LNCaP-A24 cell line was significantly suppressed by the addition of the corresponding PTHrP peptide-pulsed C1R-A24 cells, as a cold target, but this suppression was not observed with the addition of HIV peptide-pulsed C1R-A24 cells (Fig. 2C).
These results indicate that both the PTHrP 36-44 and PTHrP 102-111 peptides have the potential to induce prostate cancer-reactive CTLs from HLA-A24⁺ prostate cancer patients, and that their cytotoxicity against prostate cancer was dependent on PTHrP peptide-specific CD8⁺ T cells.

### Example 3

### Detection of IgGs reactive to the HLA-A24-binding PTHrP peptides

The inventors previously reported that IgGs reactive to CTL epitope peptides were detected in healthy donors and patients with various types of cancers [20, 21]. IgGs reactive to prostate-related antigens were also detected in healthy donors and prostate cancer patients [22-24]. It was examined, therefore, whether IgG reactive to the four PTHrP-derived peptides, PTHrP 36-44, PTHrP 102-111, PTHrP 25-34, and PTHrP 110-119, could be detected in the plasma of cancer patients and healthy donors.

Peptide-specific IgG levels in the plasma were measured by ELISA, as previously reported [20, 21]. In brief, peptide (20 µg/well)-immobilized plates were blocked with Block Ace (Yukijirushi, Tokyo, Japan) and washed with 0.05% Tween20-PBS, after which 100µl/well of plasma sample diluted with 0.05% Tween20-Block Ace was added to the plate. After 2 hours incubation at 37°C, the plates were washed and further incubated for 2 hours with a 1:1000-diluted rabbit anti-human IgG (γ-chain-specific) (DAKO, Glostrup, Denmark). The plates were washed, and then 100 µl of 1:100-diluted goat anti-rabbit IgG-conjugated horseradish peroxidase (EnVision, DAKO) was added to each well, and the plates were then incubated at room temperature for 40 min. After the plates were washed once, 100 µl/well of tetramethyl benzidine substrate solution (KPL, Guildford, UK) was added, and the reaction was stopped by the addition of 1 M phosphoric acid. The values are shown as optical density (OD) units/ml, and responses to the HIV peptide were subtracted. IgG reactive to a corresponding PTHrP peptide was judged to be positive when the difference of the OD in 1:100-diluted plasma exceeded 0.05. The results that IgG reactive to either the PTHrP102-111 or the PTHrP 109-119 peptide was detected in 8 of 10 healthy donors and in 7 of 10 prostate cancers are shown in Table 2 hereinafter. The results are also shown in Fig. 3A.

To confirm the specificity of IgG to the indicated PTHrP peptide, 100 µl of sample plasma from either HD # 1 and Pt #6 was cultured in a plate precoated with either a corresponding PTHrP peptide or an irrelevant PTHrP peptide. Thereafter, the levels of IgG reactive to the PTHrP 102-111 peptide or the PTHrP 110-119 peptide in the resulting supernatants were determined by ELISA. As a result, the levels of PTHrP peptide-specific IgG were significantly diminished by culturing the plasma in the corresponding PTHrP peptide-coated wells (Fig. 3B). This peptide-specific absorption demonstrated the validity of the present assay system.

On the other hand, IgG reactive to the PTHrP 36-44 peptide was detected in 3 of 10 healthy donors and 1 of 10 prostate cancer patients, respectively. No IgG reactive to the PTHrP 25-34 was detected in any of the healthy donors or cancer patients (Table 2 and Fig. 3A).

Table 2

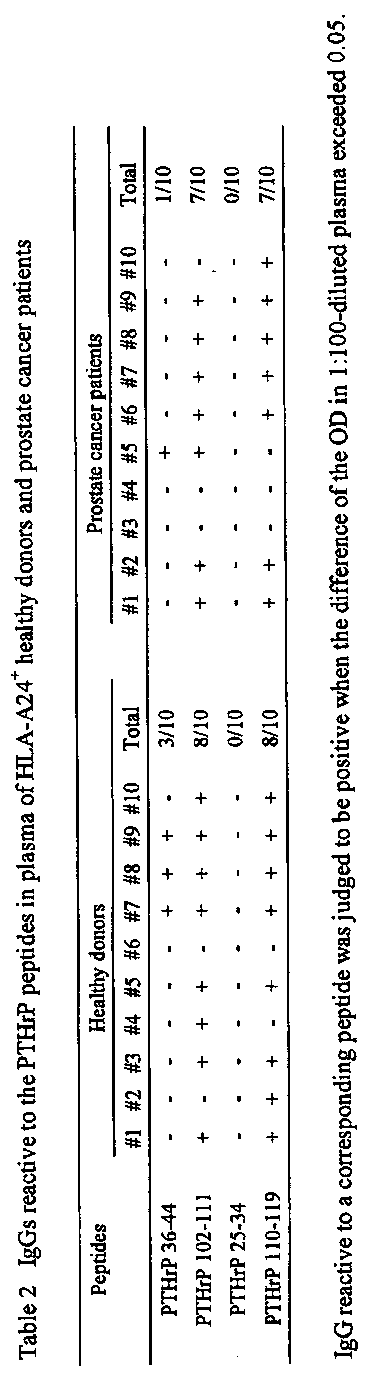

### Example 4

### Identification of candidates to generate peptide-specific CTLs from HLA-A2⁺ prostate cancer patients

### 4.1 Patients, cell lines, and peptides

In a similar way to Example 1, PTHrP-derived peptides capable of inducing peptide-specific CTLs in HLA-A2⁺ prostate cancer patients were identified. The cancer patients #1, #3, and #10 were associated with bone metastases. As the target cells, T2 cell (ATCC deposition number : CRL-1992) which is a T and B lymphoblasts-fused cell expressing HLA-A*0201 molecule (Immunogenetics, 21, 235-246, 1985) was used. Seven PTHrP-derived peptides having a HLA-A2 binding motif were prepared (listed in Table 3). Although PTHrP59-68 and PTHrP165-173 peptides had been reported to generate specific CTLs from HLA-A*0201 healthy donors [17], their predicted binding scores were less than 1 in the present study (Table 3). The numbers of the amino acid residues shared between PTH and the PTHrP peptides were as follows: PTHrP 42-51 (5), PTHrP 43-51 (4), PTHrP 51-60 (2), PTHrP 59-67 (1), PTHrP 103-111 (0).

### 4.2 Assay for peptide-specific CTLs in PBMCs

Table 3 shows that the results of peptide-specific CTL induction by those peptides in 10 HLA-A2⁺ healthy donors and 10 HLA-A2⁺ prostate cancer patients.

Table 3

**Table 3 Reactivity of PTHrP peptide-stimulated PBMCs from HLA-A2⁺ healthy donors and prostate cancer patients**

| Peptides | PTHrP 103-111 | PTHrP 59-67 | PTHrP 43-51 | PTHrP 51-60 | PTHrP 42-51 | PTHrP 165-173 | PTHrP 59-68 | Flu | EB |
|---|---|---|---|---|---|---|---|---|---|
| Ammo acid sequence | YLTQETNKV | FLHHLIAEI | LLHDKGKSI | IQDLRRAFFL | QLLHDKGKSI | TSTTSLELD | FLHHLIAEIH | GILGFVFTL | GLCTLVAML |
| Score¹ | 320 | 110 | 73 | 47 | 39 | <1 | <1 | | |
| | INF-γ production (pg/mL)² | | | | | | | | |
| Healthy donors | | | | | | | | | |
| #1 A0201 | 34 | 140 | 17 | 0 | 215 | 0 | 23 | N.D. | N.D. |
| #2 A0201 | 217 | 100 | 21 | 79 | 127 | 222 | 51 | 408 | 302 |
| #3 A0201 | 9 | 0 | 23 | 0 | 0 | 0 | 0 | 172 | 163 |
| #4 A0201 | 0 | 6 | 27 | 314 | 122 | 294 | 0 | 135 | 0 |
| #5 A0206 | 0 | 112 | 0 | 0 | 138 | 0 | 0 | N.D. | N.D. |
| #6 A0206 | 0 | 296 | 0 | 46 | 143 | 0 | 46 | 498 | 0 |
| #7 A0206 | 0 | 38 | 0 | 0 | 0 | 27 | 0 | 114 | 340 |
| #8 A0206 | 0 | 22 | 0 | 16 | 380 | 325 | 0 | 0 | 82 |
| #9 A0206 | 0 | 23 | 0 | 0 | 346 | 395 | 0 | 0 | 0 |
| #10 A0207 | 0 | 0 | 0 | 14 | 17 | 46 | 0 | 399 | 0 |
| Total | 1/10 | 4/10 | 0/10 | 2/10 | 7/10 | 4/10 | 0/10 | 6/8 | 4/8 |
| Cancer patients | | | | | | | | | |
| #1 A0206 | 144 | 118 | 40 | 55 | 155 | 202 | 31 | 0 | 24 |
| #2 A0210 | 41 | 183 | 56 | 187 | 172 | 26 | 0 | 0 | 141 |
| #3 A0210 | 57 | 129 | 185 | 34 | 179 | 60 | 48 | 166 | 0 |
| #4 A0206 | 86 | 221 | 0 | 33 | 22 | 151 | 138 | 0 | 0 |
| #5 A0206 | 0 | 0 | 39 | 0 | 153 | 103 | 154 | 0 | 0 |
| #6 A0207 | 68 | 37 | 51 | 0 | 116 | 0 | 0 | ND | ND |
| #7 A0206 | 49 | 0 | 0 | 0 | 0 | 0 | 222 | ND | ND |
| #8 A0206 | 29 | 59 | 23 | 2 | 30 | 80 | 146 | 173 | 77 |
| #9 A0201 | 56 | 0 | 14 | 67 | 0 | 0 | 43 | 172 | 163 |
| 1#10 A0207 | 0 | 49 | 0 | 21 | 0 | 24 | 112 | 27 | 0 |
| Total | 1/10 | 4/10 | 1/10 | 2/10 | 5/10 | 3/10 | 5/10 | 3/8 | 2/8 |

| | | | | | | | | | |
|---|---|---|---|---|---|---|---|---|---|
| ¹ The score represents the estimated half-time of dissociation of the PTHrP peptides binding to the HLA-A2 molecules. 2 The PBMCs of HLA-A2⁺ healthy donors and prostate cancer patients were stimulated in vitro with the PTHrP peptide indicated, as described in Examples. On the 15 th, the cultured PBMCs were tested for their reactivity to T2 cells, which were pre-pulsed with a corresponding peptide or an HIV peptide. Significant values (p<0.05 by two tailed Student's t-test) are underlined. N.D.: not done. | | | | | | | | | |

PTHrP42-51 and PTHrP59-67 peptides induced peptide-specific CTLs in 7 and 4 of 10 HLA-A2⁺ healthy donors, respectively. Although the reported PTHrP59-68 peptide is different from the PTHrP59-67 peptide in only one amino acid, no peptide-specific CTLs were induced when PBMCs from the HLA-A2⁺ healthy donors were stimulated with the PTHrP59-68 peptide. The reported PTHrP165-173 induced peptide-specific CTLs in 4 of 10 HLA-A2⁺ healthy donors. PTHrP42-51 and PTHrP59-67 peptides induced peptide-specific CTLs in 5 and 4 of 10 HLA-A2⁺ prostate cancer patients, respectively. The reported PTHrP59-68 and PTHrP165-173 peptides induced peptide-specific CTLs in 5 and 3 of 10 HLA-A2⁺ prostate cancer patients, respectively.

The most cases of successful induction of peptide-specific CTLs were different when stimulated with either the PTHrP59-67 or PTHrP59-68 peptide. In total, these findings indicate that both the PTHrP42-51 and PTHrP59-67 peptides are particularly useful new candidate peptides for generating peptide-specific CTLs from HLA-A2⁺ prostate cancer patients.

### Example 5

### Induction of prostate cancer-reactive CTLs that are HLA-A2-restricted and specific for PTHrP 42-51 or PTHrP 59-67 peptide

In a similar way to Example 2, it was examined whether or not PTHrP 42-51 and PTHrP 59-67 peptides could induce cancer-reactive CTLs in PBMCs derived from HLA-A2⁺ healthy donors and prostate cancer patients. An HLA-A2-expressing PC93 prostate cancer cell line was prepared by constitutively expressing an HLA-A*0201 molecule on a PC93 cell which was a HLA-A2-prostate cancer cell line (established by Dr. K. Ohnishi, Department of Urology, Kyoto University) (PC93-A2 cell). The expression of the HLA-A*0201 molecule on the cell line was previously reported [24]. In addition, the PC93 cells produced PTHrP at a level of 2.2 pmol/l (1 X 10⁶ cell/ml for 24 hr).

It was confirmed that PBMCs stimulated with PTHrP 42-51 and PTHrP 59-67 peptides could produce IFN-γ in response to the corresponding PTHrP peptide-pulsed T2 cells (Fig. 4A). Cytotoxicities of these peptide-stimulated PBMCs against PC93 cells, PC93-A2 cells, and PHA-stimulated T cell blasts are shown in Fig. 4B. Fig 5 shows the results of examining the cytotoxicity of the peptide-stimulated PBMCs with (A) blocking antibodies and (B) the cold inhibition assay. These results indicate that the PTHrP42-51 and PTHrP59-67 peptides have the potential to induce prostate cancer-reactive CTLs in HLA-A2⁺ prostate cancer patients, and that their cytotoxicity is dependent on peptide-specific CD8⁺ T cells.

### Example 6

### Detection of IgGs reactive to the HLA-A2-binding PTHrP peptides

In a similar way to Example 3, it was examined whether IgGs reactive to peptides of PTHrP 42-51 and PTHrP 59-67 could be detected in the plasma of 10 healthy donors and 10 cancer patients. The cut-off value at 1:100-diluted plasma was ODO.054. The result is shown in Table 4. Table 4

**Table 4 IgGs reactive to the PTHrP peptides in plasma of HLA-A2⁺ healthy donors and prostate cancer patients**

| | Peptides | | | | | | |
|---|---|---|---|---|---|---|---|
| | PTHrP 103-111 | PTHrP 59-67 | PTHrP 43-51 | PTHrP 51-60 | PTHrP 42-51 | PTHrP 165-173 | PTHrP 59-68 |
| Healthy donors | | | | | | | |
| #1 | 0.0745 | - | - | 0.0943 | - | - | 0.0855 |
| #2 | 0.092 | - | 0.102 | 0.13385 | 0.055 | - | - |
| #3 | - | - | - | - | - | - | - |
| #4 | 0.128 | - | 0.155 | - | 0.1115 | - | - |
| #5 | 0.0575 | - | - | - | 0.0615 | - | - |
| #6 | - | 0.078 | - | - | 0.0845 | 0.0865 | - |
| #7 | - | - | - | 0.0675 | 0.0874 | 0.089 | 0.119 |
| #8 | 0.0585 | - | 0.121 | - | 0.1275 | 0.193 | 0.167 |
| #9 | - | 0.112 | - | - | 0.169 | 0.0925 | 0.065 |
| #10 | - | - | - | - | 0.091 | 0.099 | 0.112 |
| Total | 5/10 | 2/10 | 3/10 | 3/10 | 8/10 | 5/10 | 5/10 |

| Cancer patients | | | | | | | |
|---|---|---|---|---|---|---|---|
| #1 | - | - | - | - | 0.0685 | - | - |
| #2 | - | 0.1225 | 0.078 | - | 0.055 | - | - |
| #3 | - | - | - | 0,0675 | 0.0715 | 0.078 | 0.0795 |
| #4 | - | - | - | - | - | - | - |
| #5 | - | - | - | - | 0.106 | - | - |
| #6 | - | - | 0.0895 | - | 0.0885 | - | - |
| #7 | 0.0655 | 0.0655 | 0.0795 | - | 0.099 | - | - |
| #8 | - | - | - | - | - | - | - |
| #9 | - | - | - | - | - | - | - |
| #10 | 0.112 | - | - | 0.0995 | 0.0995 | 0.1515 | 0.1303 |
| Total | 2/10 | 2/10 | 3/10 | 2/10 | 7/10 | 2/10 | 2/10 |

| | | | | | | | |
|---|---|---|---|---|---|---|---|
| IgG reactive to a corresponding peptide was judged to be positive when a difference of OD in a 1:100-diluted plasma was more than 0.054. | | | | | | | |

IgG reactive to the PTHrP 42-51 peptide was detected in 8 of 10 healthy donors, and in 7 of 10 prostate cancer patients. IgG reactive to the PTHrP59-67 peptide was detected in 2 of 10 healthy donors, and in 2 of 10 prostate cancer patients. The representative results of 2 healthy donors (HD # 1 and HD #6) and 2 cancer patients (Pt # 1 and Pt # 2) are shown in Fig. 6A. Peptide specificities of the IgGs were examined by absorption with the corresponding peptide and the results are shown in Fig 6B. Those results indicate that those peptides, among others PTHrP42-51 peptide, induce both of cellular and humoral immune responses in caner patients.

### INDUSTRIAL APPLICABILITY

The inventers identified new two PTHrP-derived peptides that are immunogenic in HLA-A24⁺ or HLA-A2⁺ prostate cancer patients. The frequencies of the HLA-A24 allele are relatively high throughout the world [33]. In addition, most Caucasians are HLA-A*2101 positive. On the other hand, HLA-A2 subtypes vary considerably in Japanese. PTHrP-derived peptides were prepared based on the binding motif to an HLA-A*0201 molecule, and both the T2 cells and PC93-A2 cells express HLA-A*0201 molecules. However, the peptides of the invention also induced peptide-specific CTLs from patients with other HLA-A2 subtypes, including HLA-A*0206, -A*0207, and -A*0210, as shown in Table3. The present invention increases the possibility of treating HLA-A24⁺ and HLA-A2⁺ prostate cancer patients with metastases using peptide-based immunotherapy.

### REFERENCES

1. Greenlee, R.T., Murray, T., Bolden, S., and Wingo, P. A., Cancer statistics 2000. CA Cancer J. Clin. 2000. 50:7-33.
2. Boon, T., Coulie, P.G., and Van den Eynde, B., Cancer antigens recognized by T cells. Immunol. Today 1997. 81: 267-268.
3. Rosenberg, S.A., A new era for cancer immunotheraphy based on the genes that encode cancer antigens. Immunity 1999. 10: 281-287.
4. Renkvist, N., Castelli, C., Robbins, P.F., and Parmiani, G., A listing of human cancer antigens recognized by T cells. Cancer Immunol. Immunother. 2001. 50: 3-15.
5. Nestle, F. O., Alijagic, S., Gilliet, M., Sun, Y., Grabbe, S., Dummer, R., Burg, G., and Schadendorf, D., Vaccination of melanoma patients with peptide- or tumor lysate-pulsed dendritic cells. Nature Med. 1998. 4:328-332.
6. Rosenberg, S. A., Yang, J. C., Schwartzentruber, D. J., Hwu, P., Marincola, F. M., Topalian, S. L., Restifo,, N. P., Dudley, M. E., Schwarz, S. L., Spiess, P. J., Wunderlich, J. R., Prkhurst, M. A., Kawakami, Y., Seipp, C. A., Einhorn, J. H., and White, D. E., Immunologic and therapeutic evaluation of a synthetic peptide vaccine for the treatment of patients with metastatic melanoma. Nature Med. 1998. 4:321-327.
7. Marchand, M., van Baren, N., Weynants, P., Brichard, V., Dréno, B., Tessier, M. H., Rankin, E., Parmiani, G., Arienti, F., Humblet, Y., Bourlond, A., Vanwijck, R., Lienard, D., Beauduin, M., Dietrich, P. Y., Russo, V., Kerger, J., Masucci, G., Jäger, E., De Greve, J., Atzpodien, J., Brasseur, F., Coulie, P. G., van der Bruggen, P., and Boon, T., Tumor regressions observed in patients with metastatic melanoma treated with an antigenic peptide encoded by gene MAGE-3 and presented by HLA-A1. Int. J. Cancer 1999. 80:219-230.
8. Gulley. J., Chen, A.P., Dahut, W., Arlen, P. M., Bastian, A., Steinberg, S. M., Tsang, K., Panicali, D., Poole, D., Schlom, J., and Hamilton, M. J., Phase I study of a vaccine using recombinant vaccinia virus expressing PSA (rV-PSA) in patients with metastatic androgen-independent prostate cancer. Prostate 2002. 53:109-117.
9. Murphy, G., Tjoa, B., Ragde, H., Kenny, G., and Boynton, A., Phase I clinical trial: T-cell therapy for prostate cancer using autologous dendritic cells pulsed with HLA-A0201-specific peptides from prostate-specific membrane antigen. Prostate 1996. 29:371-380 .
10. Tjoa, B.A., Simmons, S.J., Bowes, V.A., Ragde, H., Rogers, M., Elgamal, A., Kenny, G. M., Cobb, O. E., Ireton, R. C., Troychak, M. J., Salgaller, M. L., Boynton, A. L., and Murphy, G. P., Evaluation of phase I/II clinical trials in prostate cancer with dendritic cells and PSMA peptides. Prostate 1998. 36:39-44.

11. Murphy, G.P., Tjoa, B.A., Simmons, S.J., Jarisch, J., Bowes, V. A., Rogers, M., Elgamal, A., Kenny, G. M., Cobb, O. E., Ireton, R. C., Troychak, M. J., Salgaller, M. L., and Boynton, A. L., Infusion of dendritic cells pulsed with HLA-A2-specific prostate-specific membrane antigen peptides: a phase II prostate cancer vaccine trial involving patients with hormone-refractory metastatic disease. Prostate 1999. 38:73-78.
12. Small, E.J., Fratesi, P., Reese, D.M., Strang, G., Laus, R., Peshwa, M. V., and Valon, F. H., Immunotherapy of hormone-refractory prostate cancer with antigen-loaded dendritic cells. J. Clin. Oncol. 2000. 18:3894-3903.
13. Juppener, H., Abou-Samra, A. B., Freeman, M., Kong, X. F., Schipani, E., Richards, J., Kolakowski, L. F., Hock, J., Potts, J. T., Kronenberg, H. M., and Serge, G. V., AG protein-linked receptor for parathyroid hormone and parathyroid hormone-related protein. Science 1991. 254: 1024-1026.
14. Philbrick, W. M., Wysolmerski, J. J., Galbarith, S., Holt, E., Orloff, J., Yang, K. H., Vasavada, R. C., Weir, E. C., Broadus, A. E., and Stewart, A. F., Defining the roles of parathyroid hormone related protein in normal physiology. Physiol. Rev. 1996. 76: 127-173.
15. Sanders, L. J., Chattopadhyay, N., Kifor, O., Yamaguchi, T., and Brown, M. E., Ca2+-sensing receptor expression and PTHrP secretion in PC-3 human prostate cancer cells. Am. J. Physiol. Endocrinol. Metab. 2001. 281: 1267-1274.
16. Guise, T. A, Parathyroid hormone-related protein and bone metastases. Cancer 1997. 80:1572-1580.
17. Francini, G., Scardino, A., Kosmatopoulos, K., Lemonnier, F., Campoccia, G., Sabatino, M., Pozzessere, D., Petrioli, R., Lozzi, L., Neri, P., Fanetti, G., Cusi, G. M., and Correale, P., High-affinity HLA-A(*)02.01 peptides from parathyroid hormone-related protein generate in vitro and in vivo antitumor CTL response without autoimmune side effects. J. Immunol. 2002. 169: 4840-4849.
18. Parker, K. C., Bednarek, M. A., and Coligan, J. E., Scheme for ranking potential HLA-A2 binding peptides based on independent binding of individual peptide side-chains. J. Immunol. 1994. 152: 163-175.
19. Rammensee, H. G., Friege, T., and Stevanovics, S., MHC ligands and peptides motifs. Immunogenetics 1995. 41: 178-228.
20. Nakatsura, T., Senju, S., Ito, M., Nishimura, Y., and Itoh, K., Cellular and humoral immune responses to a human pancreatic cancer antigen, coactosin-like protein, originally defined by the SEREX method. Eur. J. Immunol. 2002. 32: 826-836.

21. Ohkouchi, S., Yamada, A., Imai, N., Mine, T., Harada, K., Shichijo, S., Maeda, Y., Saijyo, Y., Nukiwa, T., and Itoh, K., Non-mutated tumor rejection antigen peptides elicit type-I allergy in the majority of healthy individuals. Tissue Antigens 2002. 59: 259-272.
22. Harada, M., Kobayashi, K., Matsueda, S., Nakagawa, M., Noguchi, M., and Itoh, K., Prostate-specific antigen-derived epitopes capable of inducing cellular and humoral responses in HLA-A24+ prostate cancer patients. Prostate 2003. 57: 152-159.
23. Kobayashi, K., Noguchi, M., Itoh, K., and Harada, M., Identification of a prostate-specific membrane antigen-derived peptide capable of eliciting both cellular and humoral immune responses in HLA-A24+ prostate cancer patients. Cancer Science 2003. 94: 622-627 .
24. Matsueda, S., Kobayashi, K., Nonaka, Y., Noguchi, M., Itoh, K., and Harada, M., Identification of new prostate stem cell antigen-derived peptides immunogenic in HLA-A2+ patients with hormone-refractory prostate cancer. Cancer Immunol. Immunother. 2004, 53:479-489.
25. Harada, M., Noguchi, M., and Itoh, K., Target molecules in specific immunotherapy against prostate cancer. Int. J. Clin. Oncl. 2003. 8: 193-199.
26. Inoue, Y., Takaue, Y., Takei, M., Kato, K., Kanai, S., Harada, Y., Tobisu, K., Noguchi, M., Kakizoe, T., Itoh, K., Wakasugi, H., Induction of tumor specific cytotoxic T lymphocytes in prostate cancer using prostatic acid phosphatase derived HLA-A2402 binding peptide. J. Urol. 2001. 166:1508-1513.
27. Tian, J., Smogorzewski, M., Kedes, L., and Massry, S. G., Parathyroid hormone-parathyroid hormonerelated protein receptor messenger RNA is present in many tissues besides the kidney. Am. J. Nephrol. 1993. 13: 210-213.
28. Noguchi, M., Mine, T., Suetsugu, N., Tomiyasu, K., Suekane, S., Yamada, A., Itoh, K., and Noda, S., Induction of cellular and humoral immune responses to tumor cells and peptides in HLA-A24 positive hormone-refractory prostate cancer patients by peptide vaccination. Prostate 2003. 57: 80-92.
29. Tanaka, S., Harada, M., Mine, T., Noguchi, M., Gohara, R., Azuma, K., Tamura, M., Yamada, A., Morinaga, A., Nishikori, M., Katagiri, K., Itoh, K., Yamana, H., and Hashimoto, T., Peptide vaccination for patients with melanoma and other types of cancer based on pre-existing peptide-specific cytotoxic T lymphocyte precursors in the periphery. J. Immunother., 2003. 26: 357-366.
30. Mine, T., Gouhara R., Hida N., Imai N., Azuma K., Rikimaru T., Katagiri K., Nishikori M., Sukehiro A., Nakagawa M., Yamada A., Aizawa H., Shirouzu K., Itoh K., and Yamana H., Immunological evaluation of CTL precursor-oriented vaccines for advanced lung cancer patients. Cancer Science 2003. 94: 548-556.

31. Sato Y., Shomura H., Maeda Y., Mine T., Ueno Y., Akasaka Y., Kondo M., Takahashi S., Shinohara T., Katagiri K., Sato M., Okada S., Matsui K., Yamada A., Yamana H., Itoh K., and Todo S., Immunogical evaluation of peptide vaccination for patients with gasctirc cancer based on pre-existing cellular response to peptide. Cancer Science 2003. 94: 802-808.
32. Tsuda, N., Mochizuki, K., Harada, M., Sukehiro, A., Kawano, K., Yamada, A., Ushijima, K., Sugiyama, T., Nishida, T., Yamana, H., Itoh, K., and Kamura, T., Vaccination with pre-designated or evidence-based peptides for patients with recurrent gynecologic cancers. J. Immunother. 2004. 27: 60-72.
33. Imanishi, T., Akazawa, T., and Kimura, A., Allele and haplotype frequencies for HLA and complement loci in various ethnic groups. In Tsuji, K., Aizawa, M., Sasazuki, T. (Eds.) In: HLA 1991. Oxford Scientific Publications, vol. 1, 1992, pp1065-1220.
34. Hida, N., Maeda, Y., Katagiri, K., Takasu, H., Harada, M., and Itoh, K., A new culture protocol to detect peptide-specific cytotixic T lymphocyte precursors in the circulation. Cancer Immunol. Immunother. 2002. 51: 219-228.
35. Suva, L. J., Winslow, G. A., Wettenhall, R. E. H., Hammonds, R. G., Moseley, J. M., Diefenbach-Jagger, H., Rodda, C. P., Kemp, B. E., Rodriguez, H., Chen, E. Y., Hudson, P. J., Martin, T. J., and Wood, W. I., A parathyroid hormone-related protein implicated in malignant hypercalcemia: cloning and expression. Science 1987. 237: 893-896.
36. Harada M, Gohara R, Matsueda S, Muto A, Oda T, Iwamoto Y, Itoh K. In vivo evidence that peptide vaccination can induce HLA-DR-restricted CD4+ T cells reactive to a class I tumor peptide. J Immunol 2004; 172: 2659-2667.
37. Ito M, Shichijo S, Miyagi Y, Kobayashi T, Tsuda N, Yamada A, Saito N, Itoh K. Identification of SART3-derived peptides capable of inducing HLA-A2-restricted and tumor-specific CTLs in cancer patients with different HLA-A2 subtypes. Int J Cancer 2000; 88: 633-639.
38. Tamura M, Nishizuka S, Maeda Y, Ito M, Harashima N, Harada M, Shichijo S, Itoh K. Identification of cyclophilin B-derived peptides capable of inducing histocompatibility leukocyte antigen-A2-restricted and tumor-specific cytotoxic T lymphocytes. Jpn J Cancer Res 2001; 92: 762-767.
39. Imai N, Harashima N, Ito M, Miyagi Y, Harada M, Yamada A, Itoh K. Identification of 1ck-derived peptides capable of inducing HLA-A2-restricted and tumor-specific CTLs in cancer patients with distant metastases. Int J Cancer, 2001; 94: 237-242.

## Claims

1. A cancer antigen peptide which is a partial peptide derived from parathyroid hormone-related protein, and which is capable of binding to an HLA-A24 antigen and being recognized by cytotoxic T lymphocytes, or a derivative thereof having the functionally equivalent properties.

2. The cancer antigen peptide according to Claim 1, which is recognized by humoral immune system, or a derivative thereof having the functionally equivalent properties.

3. A cancer antigen peptide which comprises an amino acid sequence shown in any one of SEQ ID NOS: 1 to 4, or a derivative thereof which is capable of binding to HLA-A24 antigen and being recognized by cytotoxic T lymphocytes.

4. The cancer antigen peptide or derivative thereof according to Claim 3, which comprises an amino acid sequence shown in SEQ ID NO: 1 or 2.

5. A pharmaceutical composition for treating or preventing prostate cancer, which comprises as an active ingredient the cancer antigen peptide or derivative thereof according to any one of Claims 1 to 4.

6. The pharmaceutical composition according to Claim 5, wherein the prostate cancer is associated with bone metastases.

7. A pharmaceutical composition for treating or preventing metastatic bone lesion, which comprises as an active ingredient the cancer antigen peptide or derivative thereof according to any one of Claims 1 to 4.

8. The pharmaceutical composition according to Claim 7, wherein the metastatic bone lesion are accompanied by prostate cancer.

9. A cytotoxic T lymphocyte which specifically recognizes a complex between an HLA-A24 antigen and the cancer antigen peptide or derivative thereof according to any one of Claims 1 to 4.

10. A pharmaceutical composition for treating prostate cancer, which comprises as an active ingredient the cytotoxic T lymphocyte of Claim 9.

11. The pharmaceutical composition according to Claim 10, wherein the prostate cancer is associated with bone metastases.

12. An antigen presenting cell which presents a complex between HLA-A24 antigen and the cancer antigen peptide or derivative thereof according to any one of Claims 1 to 4 on the cell surface.

13. A cancer antigen peptide which comprises an amino acid sequence shown in any one of SEQ ID NOS: 5 to 9 or a derivative thereof which is capable of binding to an HLA-A2 antigen and being recognized by cytotoxic T lymphocytes.

14. The cancer antigen peptide or derivative thereof according to Claim 13, which is the cancer antigen peptide comprising an amino acid sequence shown in SEQ ID NO: 5 or 6 or the derivative thereof.

15. A pharmaceutical composition for treating or preventing prostate cancer, which comprises as an active ingredient the cancer antigen peptide or derivative thereof according to Claims 13 or 14.

16. The pharmaceutical composition according to Claim 15, wherein the prostate cancer is associated with bone metastases.

17. A pharmaceutical composition for treating or preventing metastatic bone lesion, which comprises as an active ingredient the cancer antigen peptide or derivative thereof according to Claims 13 or 14.

18. The pharmaceutical composition according to Claim 17, wherein the metastatic bone lesion are accompanied by prostate cancer.

19. A cytotoxic T lymphocyte that specifically recognizes a complex between an HLA-A2 antigen and the cancer antigen peptide or derivative thereof according to Claims 13 or 14.

20. A pharmaceutical composition for treating prostate cancer, which comprises as an active ingredient the cytotoxic T lymphocyte of Claim 19.

21. The pharmaceutical composition according to Claim 20, wherein the prostate cancer is associated with bone metastases.

22. An antigen presenting cell which presents a complex between an HLA-A2 antigen and the cancer antigen peptide or derivative thereof according to Claims 13 or 14 on the cell surface.

23. A method for treating or preventing prostate cancer, which comprises administering to a patient in need thereof the cancer antigen peptide or derivative thereof according to any one of Claims 1 to 4.

24. A method for treating or preventing metastatic bone lesion, which comprises administering to a patient in need thereof the cancer antigen peptide or derivative thereof according to any one of Claims 1 to 4.

25. A method for treating or preventing prostate cancer, which comprises administering to a patient in need thereof the cancer antigen peptide or derivative thereof according to Claims 13 or 14.

26. A method for treating or preventing metastatic bone lesion, which comprises administering to a patient in need thereof the cancer antigen peptide or derivative thereof according to Claims 13 or 14.

27. Use of the cancer antigen peptide or derivative thereof according to any one of Claims 1 to 4 for manufacturing a pharmaceutical composition for treating or preventing prostate cancer.

28. Use of the cancer antigen peptide or derivative thereof according to any one of Claims 1 to 4 for manufacturing a pharmaceutical composition for treating or preventing metastatic bone lesion.

29. Use of the cancer antigen peptide or derivative thereof according to Claims 13 or 14 for manufacturing a pharmaceutical composition for treating or preventing prostate cancer.

30. Use of the cancer antigen peptide or derivative thereof according to Claims 13 or 14 for manufacturing a pharmaceutical composition for treating or preventing metastatic bone lesion.
